# FASCICULE DE BREVET EUROPEEN

(11) **EP 1 465 594 B1**
(45) Date de publication et mention de la délivrance du brevet: **21.07.2010**
(21) Numéro de dépôt: 02804932.8
(22) Date de dépôt: 17.12.2002
(51) Int. Cl.: A61K 9/00, A61K 47/38

(54) **FORME GALENIQUE SOLIDE POUR L ADMINISTRATION OCULAIRE DE PRINCIPE ACTIF, INSERT OPHTALMIQUE SOLIDE ET SOLUBLE ET PROCEDE DE FABRICATION D'UN TEL INSERT**
"FESTE DOSIERUNGSFORM ZUR OKULAREN APPLIKATION EINER WIRKSUBSTANZ, LÖSLICHE, FESTE OPHTHALMISCHE EINLAGE SOWIE HERSTELLUNGSVERFAHREN DAFÜR"
SOLID DOSAGE FORM FOR THE OCULAR ADMINISTRATION OF AN ACTIVE PRINCIPLE, A SOLUBLE, SOLID OPHTHALMIC INSERT AND THE PRODUCTION METHOD THEREOF

(30) Priorité: 18.12.2001 FR 0116377
(43) Date de publication de la demande: 13.10.2004
(73) Titulaire: Ioltechnologie-Production, 17180 Perigny (FR)
(72) Inventeur: EL MESKI, Said, 21000 DIJON (FR); TOURRETTE, Philippe, F-17220 La Jarrie (FR); AIACHE, Jean-Marc, F-63000 Clermont-Ferrand (FR); DI BATTISTA, Marie, F-33500 Arveyres (FR); SERPIN, Gilbert, F-63000 Clermont-Ferrand (FR)
(74) Mandataire: Perin, Georges
(86) Numéro de dépôt international: PCT/FR2002/004384
(87) Numéro de publication internationale: WO 2003/051330

(56) Documents cités:
- EP-A- 0 316 838
- EP-A- 0 561 695
- WO-A-94/05257
- US-A- 4 179 497
- US-A- 4 343 787

## Description

La présente invention concerne une forme galénique solide pour l'administration oculaire de principe actif, un insert ophtalmique solide et soluble obtenu à partir de cette forme, ainsi qu'un procédé de fabrication d'un tel insert.

La forme galénique de l'invention peut être utilisée indifféremment en médecine humaine ou vétérinaire pour une action locale ou systémique.

De nombreuses compositions pharmaceutiques sont utilisées dans le traitement des troubles oculaires. Ces compositions sont en général des formes liquides, des gels ou des pommades. Du fait de la sécrétion et du drainage du liquide lacrymal, ces formes ne permettent pas un temps de contact suffisant pour une pénétration satisfaisante du ou des principes actifs au niveau de la cornée.

Depuis 1973, des formes solides sèches de type insert à déposer dans le cul-de-sac conjonctival sont apparues. Ces inserts sont généralement composés d'une matrice de polymères insolubles destinés à libérer le principe actif de manière prolongée. Ils doivent donc être retirés du cul-de-sac conjonctival après un temps de contact suffisant. De telles formes sont particulièrement indiquées lorsque le traitement prescrit doit être de longue durée ou qu'il nécessite un temps de contact prolongé. L'emploi de ce type d'insert est également recherché pour l'administration d'une dose précise d'un principe actif.

De tels inserts insolubles peuvent parfois présenter l'inconvénient de libérer lentement les principes actifs retenus dans la matrice. Ils peuvent également être incompatibles lorsque l'on recherche une action immédiate.

Du fait de cette matrice insoluble, la dose de principe actif doit être augmentée et le profil de libération n'est pas toujours contrôlé car une certaine quantité de principe actif n'est pas libérée par la matrice polymérique. Un autre inconvénient de ces formes est qu'elles doivent être retirées après utilisation avec un risque de rupture de l'insert au moment du retrait.

Des inserts oculaires solubles biodégradables ont été mis au point à partir de polymères biocompatibles hydrosolubles. Ces inserts sont également destinés à une libération prolongée et contrôlée d'une substance médicamenteuse par solubilisation ou gonflement lent et progressif des polymères utilisés.

US-A-4 179 497 décrit des inserts ophtalmiques de pilocarpine à base d'une matrice d'hydoxypropylcellulose (HPC), réalisés par extrusion ou par compression-moulage à chaud. Les inserts obtenus, qui mettent à profit les propriétés thermoplastiques de l'HPC, sont mous et flexibles après compression. Administrés dans l'oeil, ils se ramollissent puis se dissolvent lentement. La mise en forme à chaud peut dégrader les principes actifs sensibles.

Il serait donc souhaitable de disposer de formes galéniques solides pour l'administration oculaire de principes actifs permettant une libération rapide de ce dernier.

Il serait également souhaitable de disposer de formes galéniques solides pour l'administration oculaire de principes actifs permettant une libération contrôlée, ainsi que d'inserts ophtalmiques solubles permettant une libération rapide et contrôlée de principes actifs.

Il est, de manière générale, toujours souhaitable de limiter au maximum le nombre total d'ingrédients, par exemple pour des questions de coûts ou d'interaction entre les différents constituants.

C'est pourquoi la présente demande a pour objet une forme galénique solide pour l'administration oculaire de principes actifs de l'invention **caractérisée en ce qu**'elle comporte au moins un excipient hydrosoluble et biocompatible à un usage ophtalmique et en ce qu'elle est obtenue par un procédé choisi parmi la compression et la lyophilisation, de telle sorte qu'elle puisse se déliter dans le cul-de-sac conjonctival, et notamment une forme galénique solide pour l'administration oculaire d'un principe actif, **caractérisée en ce qu**'elle comporte au moins un excipient hydrosoluble et biocompatible à un usage ophtalmique et en ce qu'elle est obtenue par un procédé choisi parmi :
- la compression directe,
- la compression par voie sèche,
- la compression par voie humide,
- la compression d'un lyophilisat,
la compression étant opérée à une température inférieure à 45°C, et
- la lyophilisation,
de telle sorte qu'elle puisse se déliter et libérer le principe actif dans le cul-de-sac conjonctival.

La forme galénique suivant l'invention est sèche et rigide.

Par "principe actif" on entend, au sens de la présente invention, non seulement un composé ayant une action pharmacologique mais aussi un composé n'ayant pas d'action pharmacologique mais par exemple une action physique, utilisable à titre illustratif dans le syndrome de l'oeil sec, ou mécanique.

Par "hydrosoluble", l'on entend que l'excipient est directement soluble ou partiellement soluble dans l'eau, sans passage par un stade de gel.

L'excipient hydrosoluble et biocompatible à un usage ophtalmique est avantageusement choisi parmi les polymères hydrosolubles.

L'excipient hydrosoluble et biocompatible à un usage ophtalmique est par exemple choisi parmi les hydrates de carbone, les gommes, les sels de sodium, les sels de calcium, les sels de magnésium, les maltodextrines, les dérivés cellulosiques, les chitosans, les amidons déstructurés, les amidons partiellement hydrolysés, les amidons réticulés, les alcools polyvinyliques et les polymères d'acides acryliques.

Les dérivés cellulosiques sont de préférence choisis parmi les hydroxyalkylcelluloses, par exemple l'hydroxypropylcellulose.

L'excipient hydrosoluble et biocompatible à un usage ophtalmique peut être présent à une concentration pouvant atteindre 99,9 % en poids, de préférence comprise entre environ 40 et environ 99,5 %, notamment entre 50 % et 99 %, particulièrement entre 60 % et 95 %.

Selon l'invention, la forme galénique peut contenir en outre des excipients conventionnels de formes ophtalmiques choisis parmi les lubrifiants solubles, les agents de désintégration, les agents d'écoulement, les agents d'enrobage polymériques et les liants.

Le lubrifiant soluble est par exemple choisi parmi les sucro esters, le polyéthylène glycol, la leucine et le borate de sodium.

L'agent de désintégration est par exemple choisi parmi les dérivés cellulosiques, la croscarmellose sodique, la crospovidone et la povidone.

Les dérivés cellulosiques sont par exemple la carboxyméthylcellulose sodique.

Les agents d'enrobage polymériques sont en général choisis parmi les dérivés thermoplastiques de cellulose. Les dérivés thermoplastiques de cellulose sont par exemple l'éthylcellulose et l'acétate de cellulose. Ils doivent être solubles dans les larmes.

Le nombre d'excipients que renferme une forme galénique selon l'invention est avantageusement inférieur à 5, de préférence inférieur à 4, notamment inférieur à 3 ; particulièrement il n'y a qu'un seul excipient.

Dans des conditions préférentielles de mise en oeuvre de l'invention, les formes galéniques ci-dessus sont substantiellement dépourvues d'agents plastifiants comme le polyéthylène glycol, la glycérine ou un hydroxypropyl sucrose.

Dans d'autres conditions préférentielles de mise en oeuvre de l'invention, les formes galéniques ci-dessus sont substantiellement dépourvues d'agent conservateur.

Par " substantiellement dépourvues", l'on entend que la quantité pondérale du composé en question est inférieure à 1%, notamment inférieure à 0,5% du poids total de la forme terminée. Dans des conditions tout particulièrement préférées le produit en question est totalement absent de la forme galénique ci-dessus.

La forme galénique de l'invention peut en outre contenir une substance pharmacologiquement active ou non pharmacologiquement active mais par exemple à action physique ou mécanique.

La substance pharmacologiquement active est par exemple choisie parmi les agents anesthésiques comme la tétracaïne, la lidocaïne ou la bupivacaïne, les agents antiallergiques comme le chromoglycate de sodium, les anti-inflammatoires corticoïdes comme le sulfate de dexaméthasone ou d'autres sels de dexaméthasone, les anti-inflammatoires non stéroidiens comme le diclofénac, l'indométacine ou l'ibuprofène, les antibiotiques comme la gentamicine ou la tobramycine, les agents antibactériens ou antiviraux comme l'aciclovir ou le ganciclovir, les mydriatiques comme la phényléphrine, tropicamide, atropine, les agents antiglaucomateux comme la pilocarpine, le timolol, les prostaglandines. On peut citer aussi les agents antiseptiques comme la ciclosporine, les agents cicatrisants, les produits de diagnostic, les suppléants lacrymaux, les agents vasoconstricteurs, les agents myotiques, les agents antimycosiques, et les agents antiphlogistiques.

La substance pharmacologiquement active peut être choisie pour une action à visée locale ou pour une action à visée systémique.

Une substance pharmacologiquement inactive pourra être par exemple l'hydroxypropylcellulose permettant de traiter le syndrome de l'oeil sec.

L'homme du métier déterminera aisément la quantité de substance pharmacologiquement active en fonction de la nature de la substance et de l'effet thérapeutique désiré.

La forme galénique de l'invention est avantageusement stérile et conditionnée individuellement ou en multidoses de préférence dans un dispositif facilitant son administration.

Le ou les excipients et la mise en oeuvre du procédé, par exemple la force de compression, seront avantageusement choisis par l'homme de l'art pour conférer à une forme galénique selon l'invention un temps de délitement inférieur à deux heures, de préférence inférieur à une heure, notamment inférieur à 45 mn, particulièrement inférieur à 30 mn, tout particulièrement inférieur à 15 mn. Le délitement pourra par exemple intervenir en 2 mn.

Par exemple, en ajoutant un agent de désintégration, on obtient un raccourcissement de la durée de délitement.

Les formes galéniques solides pour l'administration oculaire objet de la présente invention possèdent de très intéressantes propriétés physico-chimiques. Elles permettent notamment une libération rapide du ou des principes actifs. Elles permettent aussi une libération contrôlée de ces principes actifs.

Ces propriétés sont illustrées ci-après dans la partie expérimentale. Elles justifient l'utilisation des formes galéniques solides pour l'administration oculaire ci-dessus décrites à titre de formes de transport notamment de médicaments.

Les formes galéniques solides pour l'administration oculaire selon la présente invention trouvent leur emploi par exemple dans le traitement tant curatif que préventif de l'inflammation oculaire, du syndrome de l'oeil sec, de l'allergie oculaire, ainsi qu'en anesthésie.

La forme galénique de l'invention peut être utilisée indifféremment en médecine humaine ou vétérinaire, pour une action locale ou systémique.

C'est pourquoi l'invention a aussi pour objet un insert ophtalmique constitué par une forme galénique telle que décrite ci-dessus et ayant des dimensions appropriées pour une administration oculaire.

La forme galénique constituant l'insert ophtalmique a avantageusement une longueur comprise entre environ 1 mm et environ 10 mm, par exemple comprise entre environ 2 mm et environ 5 mm pour un insert oblong. Un insert de forme circulaire aura par exemple un diamètre pouvant aller jusqu'à environ 10 mm, notamment compris entre environ 1 et environ 4 mm.

La forme galénique de l'invention peut notamment être préparée :
- par compression directe,
- par compression par voie sèche,
- par compression par voie humide,
- par compression d'un lyophilisat. On peut à cette fin préparer un lyophilisat à base d'un mélange, notamment à parties égales, entre un principe actif et du mannitol, mélanger ce lyophilisat par exemple avec de l'hydroxypropylcellulose et du PEG 6000, de préférence après homogénéisation, mélanger l'ensemble avec un excipient de compression directe comme le mannitol, et comprimer à l'aide d'un poinçon approprié,
- par réalisation d'un lyophilisat.

Pour préparer une forme galénique selon l'invention, on peut notamment procéder comme suit :
(a) mélange des différents constituants sous forme de poudre et compression directe du mélange, le principe actif éventuel et les excipients étant tamisés sur un tamis de maille comprise en général entre 0,100 et 0,630 mm ;
   Avant compression directe on peut rajouter par exemple un agent humidifiant tel que l'acide hyaluronique, un agent lubrifiant ou alors faire des mélanges à base de lactose, des sucres ou saccharose tels que le Microcristal 120® ou Compressuc®, de granulométrie différente.
(b) granulation ou enrobage de tout ou partie des excipients et compression ;
(c) dissolution du principe actif dans un solvant tel qu'une solution aqueuse ou saline et de préférence une solution à base d'un saccharide comme le mannitol ou le sorbitol suivie de l'évaporation du solvant selon la technique de lyophilisation.

On opère la compression à une température inférieure à 45°C, de préférence inférieure à 35°C, notamment inférieure à 30°C, particulièrement inférieure à 25°C et tout particulièrement à température ambiante.

Dans certains cas, le procédé peut être réalisé à froid (4°C par exemple) pour éviter une éventuelle dégradation d'un principe actif quand il est thermolabile.

Les conditions préférentielles de mise en oeuvre des formes galéniques solides pour l'administration oculaire ci-dessus décrites s'appliquent également aux autres objets de l'invention visés ci-dessus.

La présente demande a enfin pour objet l'utilisation d'une forme galénique solide comportant au moins un excipient hydrosoluble et biocompatible à un usage ophtalmique et obtenue par les procédés ci-dessus, pour se déliter dans le cul-de-sac conjonctival, pour l'administration oculaire d'un principe actif.

L'invention sera à présent illustrée décrite par les exemples suivants.

### Exemple 1

On prépare une forme galénique selon l'invention sous forme d'un insert ophtalmique contenant de la tétracaïne chlorhydrate comme substance pharmacologiquement active incorporée dans le mélange de poudres obtenu à partir des composants suivants du Tableau I.

**Tableau I.**

| Composant | Pourcentage en poids |
|---|---|
| Tétracaïne chlorhydrate | 22 |
| Lactose pour compression directe | 78 |

Le lactose pour compression directe utilisé est le lactose commercialisé par la société BASF sous la dénomination Ludipress® LCE.

Le principe actif et l'excipient sont tamisés sur un tamis de maille 0,400 mm. Ils sont ensuite pesés et mélangés pendant 5 à 10 minutes.

Un insert ophtalmique est ensuite obtenu en effectuant une compression directe du mélange obtenu, sur une machine rotative équipée de 12 poinçons de forme oblongue ayant 4,3 mm de longueur et 2,3 mm de largueur.

Le temps de dissolution de l'insert ophtalmique obtenu est de 4 minutes.

### Exemple 2

On prépare une forme galénique à partir des composants du Tableau II selon le mode opératoire de l'Exemple 1.

**Tableau II**

| Composant | Pourcentage en pds |
|---|---|
| Tétracaïne chlorhydrate | 9 |
| Lactose pour compression directe | 86 |
| Crospovidone | 5 |

Le lactose pour compression directe utilisé est le lactose commercialisé par la société BASF sous la dénomination Ludipress® LCE.

La Crospovidone utilisée comme agent de désintégration, est commercialisée par la société BASF sous la dénomination Kollidon® CL-M.

Un insert ophtalmique est obtenu comme dans l'Exemple 1.

Le temps de dissolution de l'insert ophtalmique obtenu est de 2,5 minutes

### Exemple 3

On prépare une forme galénique à partir des composants du Tableau III selon le mode opératoire de l'Exemple 1.

**Tableau III**

| Composant | Pourcentage en pds |
|---|---|
| Tétracaïne chlorhydrate | 10 |
| Lactose pour compression directe | 78 |
| Crospovidone | 12 |

Le lactose pour compression directe utilisé est le lactose commercialisé par la société BASF sous la dénomination Ludipress®.

Un insert ophtalmique est obtenu comme dans l'Exemple 1 ou 2.

Le temps de dissolution de l'insert ophtalmique obtenu est de 4,5 minutes

### Exemple 4

On prépare une forme galénique à partir des composants du Tableau IV selon le mode opératoire de l'Exemple 1.

**Tableau IV**

| Composants | Pourcentage |
|---|---|
| Lidocaïne chlorhydrate | 3,3 |
| Polyéthylène glycol 6000 | 10 |
| Lactose pour compression directe | 86,7 |

Le lactose pour compression directe utilisé est le lactose commercialisé par la société BASF sous la dénomination Ludipress® LCE.

Un insert ophtalmique est obtenu comme dans l'Exemple 1 ou 2.

Le temps de dissolution de l'insert ophtalmique obtenu est de 2 minutes

### Exemple 5

On prépare une forme galénique à partir des composants du Tableau V selon le mode opératoire de l'Exemple 1.

**Tableau V**

| Composants | Pourcentage |
|---|---|
| Tétracaïne chlorhydrate | 3,3 |
| Hydroxyéthylcellulose | 5 |
| Polyéthylène glycol 6000 | 10 |
| Lactose pour compression directe | 81,7 |

Le lactose pour compression directe utilisé est le lactose commercialisé par la société BASF sous la dénomination Ludipress® LCE.

Un insert ophtalmique est obtenu comme dans l'Exemple 1 ou 2.

Le temps de dissolution de l'insert ophtalmique obtenu est de 3 minutes

### Exemple 6

On prépare une forme galénique à partir des composants du Tableau VI selon le mode opératoire de l'Exemple 1.

**Tableau VI**

| Composants | Pourcentage |
|---|---|
| Tétracaïne chlorhydrate | 11,3 |
| Hydroxypropylméthylcellulose | 5 |
| Sucroesters | 2 |
| Lactose pour compression directe | 81,7 |

Le lactose pour compression directe utilisé est le lactose commercialisé par la société BASF sous la dénomination Ludipress® LCE.

Un insert ophtalmique est obtenu comme dans l'Exemple 1 ou 2.

Le temps de dissolution de l'insert ophtalmique obtenu est de 4 minutes 50 secondes.

Il est évident pour l'homme de l'art que le même principe actif, un anesthésique, a été utilisé uniquement pour permettre des comparaisons des temps de dissolution mais que tout principe actif utilisable en ophtalmologie peut être intégré dans la composition selon l'invention.

### Exemple 7 : forme galénique préparée par lyophilisation / compression

On prépare une forme galénique selon l'invention à partir des composants du tableau suivant :

| Composants | Pourcentage en poids |
|---|---|
| Mélange lyophilisé (lactoferrine + mannitol) | 46 |
| HPC (Klucel® EP Pharm 100) | 31,2 |
| Mannitol pour compression directe (Pearlitol® 100 SD) | 17 |
| PEG 6000 | 5,8 |

- On réalise un lyophilisat à base d'un mélange à parties égales entre le principe actif (lactoferrine) et le mannitol,
- on homogénéise,
- on mélange avec l'HPC et le PEG 6000,
- on mélange avec le mannitol pour compression directe,
- on procède à la compression pour obtenir un insert selon l'invention.
- on effectue un conditionnement primaire puis secondaire.

L'insert ophtalmique obtenu est une forme galénique d'un poids moyen de 12 mg et d'une résistance à la rupture (dureté de 40N évaluée dans un essai de dureté selon la pharmacopée européenne).

Le mannitol par compression directe est rajouté pour améliorer l'écoulement de la poudre et obtenir une meilleure compression du mélange de lyophilisat obtenu.

### Exemple 8 : formule galénique préparée par compression après granulation par voie humide

On prépare une forme galénique à partir des composants du Tableau suivant:

| Composants | Pourcentage en poids |
|---|---|
| Diclofénac | 1,3 |
| HPMC (Métolose® 90 SH) 1^{ère} portion | 20 |
| HPMC (Métolose® 90 SH) 2^{éme} portion | 1,3 |
| Lactose pour compression directe | 77,4 |

- on mélange le diclofénac et la première portion de l'HPMC,
- on procède à une granulation humide avec un mélange eau / alcool éthylique,
- on sèche pendant 2 heures à 42°C et on tamise,
- on mélange les granulés tamisés avec la deuxième portion de l'HPMC,
- on ajoute le lactose pour compression directe, et procède à la compression des 500 g de granulés obtenus pour obtenir des composés d'un poids moyen de 12 mg se délitant en 1 H 30.

En faisant varier les pourcentages des composants, on peut varier les temps de dissolution de l'insert obtenu. Ce temps peut d'être environ 20 mn, parfois entre 1 H 30 et 2 heures, mais il peut varier et atteindre 8 heures, voire un temps de délitement compris entre 12 et 24 heures.

### Exemple 9 : formule galénique préparée par compression après granulation par voie humide

On prépare une forme galénique à partir des composants du Tableau suivant :

| Composants | Pourcentage en poids |
|---|---|
| Diclofénac | 1,2 |
| Leucine (Fluka) | 20 |
| Leucine (sigma) | 33,8 |
| Leucine (sigma) (phase externe) | 1,2 |
| Lactose pour compression directe | 38,8 |

- on mélange le diclofénac et l'HPMC,
- puis on ajoute la leucine Fluka et la leucine Sigma,
- on procède à une granulation humide du mélange obtenu avec un mélange eau / alcool éthylique,
- on sèche le grain pendant 2 heures à 42°C et on tamise,
- on mélange le grain obtenu avec la leucine restante mise en phase externe,
- on ajoute le lactose pour compression directe,
- on procède à la compression des 500 g de granulés obtenus en réglant la force de compression pour que l'insert ophtalmique fabriqué, d'un poids moyen de 12 mg, permette une libération du diclofénac sur au moins huit heures.

## Revendications

1. Forme galénique solide pour l'administration oculaire d'un principe actif, **caractérisée en ce que** :
- elle comporte au moins un excipient hydrosoluble et biocompatible à un usage ophtalmique ;
- elle est sèche et rigide ;
- elle est obtenue par un procédé choisi parmi :
la compression directe,
la compression par voie sèche,
la compression par humide,
la compression d'un lyophilisat,
la compression étant opérée à une température inférieure à 45°C, et la lyophilisation ; et
- elle présente un temps de délitement libérant le principe actif dans le cul-de-sac conjonctival inférieur à 2 heures.

2. Forme galénique selon la revendication 1, **caractérisée en ce que** l'excipient hydrosoluble est choisi parmi les polymères hydrosolubles et biocompatibles à un usage ophtalmique.

3. Forme galénique selon la revendication 2, **caractérisée en ce que** l'excipient hydrosoluble biocompatible est choisi parmi les hydrates de carbone, les gommes, les sels de sodium, les sels de calcium, les sels de magnésium, les maltodextrines, les dérivés cellulosiques, les chitosans, les amidons déstructurés, les amidons partiellement hydrolysés, les amidons réticulés, les alcools polyvinyliques et les polymères d'acides acryliques.

4. Forme galénique selon la revendication 1 ou 2, **caractérisée en ce que** l'excipient hydrosoluble et biocompatible à un usage ophtalmique est un dérivé cellulosique choisi parmi les hydroxyalkylcelluloses.

5. Forme galénique selon l'une quelconque des revendications 1 à 4, **caractérisée en ce que** l'excipient hydrosoluble et biocompatible à un usage ophtalmique est l'hydroxypropylcellulose.

6. Forme galénique selon l'une des revendications 1 à 3, **caractérisée en ce que** l'excipient hydrosoluble et biocompatible à un usage ophtalmique est choisi parmi les hydrates de carbone.

7. Forme galénique selon l'une des revendications 1 à 6, **caractérisée en ce que** l'excipient hydrosoluble est présent à une concentration comprise entre environ 40 et environ 99,5% en poids.

8. Forme galénique selon l'une des revendications 1 à 7, **caractérisée en ce qu'**elle contient en outre au moins un excipient choisi parmi les lubrifiants solubles, les agents de désintégration, les agents d'écoulement, les agents d'enrobage polymériques et les liants.

9. Forme galénique selon l'une des revendications 1 à 8, **caractérisée en ce qu'**elle contient en outre au moins un lubrifiant soluble choisi parmi les sucro esters, le polyéthylène glycol, la leucine et le borate de sodium.

10. Forme galénique selon l'une des revendications 1 à 9, **caractérisée ne ce qu**'elle contient en outre au moins un agent de désintégration choisi parmi les dérivés cellulosiques, la croscarmellose sodique, la crospovidone et la povidone.

11. Forme galénique selon la revendication 10, **caractérisée** ne ce que le dérivé cellulosique est la carboxyméthylcellulose sodique.

12. Forme galénique selon l'une des revendications 1 à 11, **caractérisée en ce qu'**elle contient en outre au moins un agent d'enrobage polymérique choisi parmi les dérivés thermoplastiques de cellulose.

13. Forme galénique selon l'une des revendications 1 à 12, **caractérisée en ce qu'**elle contient en outre au moins un dérivé thermoplastique de cellulose choisi parmi l'éthylcellulose et l'acétate de cellulose.

14. Forme galénique selon l'une quelconque des revendications 1 à 13, **caractérisée en ce qu'**elle contient une substance pharmacologiquement active.

15. Forme galénique selon la revendication 14, **caractérisés en ce que** la substance pharmacologiquement active est choisie pour une action à visée locale.

16. Forme galénique selon la revendication 14, **caractérisée ne ce que** la substance pharmacologiquement active est choisie pour une action à visée systémique.

17. Forme galénique selon l'une quelconque des revendications 1 à 16, **caractérisée en ce qu'**elle est stérile et conditionnée individuellement ou en multidoses.

18. Utilisation d'une forme galénique solide selon l'une quelconque des revendications 1 à 17 pour la préparation d'un insert ophtalmique pour l'administration oculaire d'un principe actif par délitement dans le cul-de-sac conjonctival.

19. Utilisation selon la revendication 18, **caractérisée en ce que** le temps de délitement est inférieur à 2 heures.

20. Insert ophtalmique constitué par une forme galénique selon l'une quelconque des revendications 1 à 17, **caractérisé en ce que** ladite forme galénique a des dimensions appropriées pour une administration oculaire.

21. Insert ophtalmique selon la revendication 20, **caractérisé en ce que** la forme galénique a une longueur comprise entre environ 1 mm et environ 10 mm ou un diamètre pouvant aller jusqu'à environ 10 mm.

22. Insert ophtalmique selon la revendication 20 ou 21, **caractérisé en ce que** la forme galénique a une longueur comprise entre environ 2 mm et environ 5 mm ou un diamètre compris entre environ 1 et environ 4 mm.

## Claims

1. Solid dosage form for the ocular administration of an active principle, **characterised in that**:
- it comprises at least one water-soluble excipient biocompatible with an ophthalmic use;
- it is dry and rigid;
- it is obtained by a process chosen from:
direct compression,
dry compression,
wet compression,
compression of a lyophilisate,
the compression being carried out at a temperature of less than 45°C, and
lyophilisation; and
- the time taken for it to disintegrate and release the active principle in the conjunctival cul-de-sac is less than 2 hours.

2. Dosage form according to claim 1, **characterised in that** the water-soluble excipient is chosen from water-soluble polymers biocompatible with an ophthalmic use.

3. Dosage form according to claim 2, **characterised in that** the biocompatible water-soluble excipient is chosen from carbohydrates, gums, sodium salts, calcium salts, magnesium salts, maltodextrins, cellulose derivatives, chitosans, destructured starches, partially hydrolysed starches, crosslinked starches, polyvinyl alcohols and acrylic acid polymers.

4. Dosage form according to claim 1 or 2, **characterised in that** the water-soluble excipient biocompatible with an ophthalmic use is a cellulose derivative chosen from hydroxyalkylcelluloses.

5. Dosage form according to any one of claims 1 to 4, **characterised in that** the water-soluble excipient biocompatible with an ophthalmic use is hydroxypropylcellulose.

6. Dosage form according to one of claims 1 to 3, **characterised in that** the water-soluble excipient biocompatible with an ophthalmic use is chosen from carbohydrates.

7. Dosage form according to one of claims 1 to 6, **characterised in that** the water-soluble excipient is present at a concentration of between approximately 40 and approximately 99.5% by weight.

8. Dosage form according to one of claims 1 to 7, **characterised in that** it additionally comprises at least one excipient chosen from soluble lubricants, disintegrating agents, flow agents, polymeric coating agents and binders.

9. Dosage form according to one of claims 1 to 8, **characterised in that** it additionally comprises at least one soluble lubricant chosen from sucroesters, polyethylene glycol, leucine and sodium borate.

10. Dosage form according to one of claims 1 to 9, **characterised in that** it additionally comprises at least one disintegrating agent chosen from cellulose derivatives, croscarmellose sodium, crospovidone and povidone.

11. Dosage form according to claim 10, **characterised in that** the cellulose derivative is sodium carboxymethylcellulose.

12. Dosage form according to one of claims 1 to 11, **characterised in that** it additionally comprises at least one polymeric coating agent chosen from thermoplastic cellulose derivatives.

13. Dosage form according to one of claims 1 to 12, **characterised in that** it additionally comprises at least one thermoplastic cellulose derivative chosen from ethylcellulose and cellulose acetate.

14. Dosage form according to any one of claims 1 to 13, **characterised in that** it comprises a pharmacologically active substance.

15. Dosage form according to claim 14, **characterised in that** the pharmacologically active substance is chosen in order to have a local action.

16. Dosage form according to claim 14, **characterised in that** the pharmacologically active substance is chosen in order to have a systemic action.

17. Dosage form according to any one of claims 1 to 16, **characterised in that** it is sterile and packaged individually or als multidoses.

18. Use of a solid dosage form according to any one of claims 1 to 17 in order to prepare an ophthalmic insert for the ocular administration of an active principle which disintegrates in the conjunctival cul-de-sac.

19. Use according to claim 18, **characterised in that** the disintegration time is less than 2 hours.

20. Ophthalmic insert composed of a dosage form according to any one of claims 1 to 17, **characterised in that** said dosage form has dimensions appropriate for an ocular administration.

21. Ophthalmic insert according to claim 20, **characterised in that** the dosage form has a length of between approximately 1 mm and approximately 10 mm or a diameter which can range up to approximately 10 mm.

22. Ophthalmic insert according to claim 20 or 21, **characterised in that** the dosage form has a length of between approximately 2 mm and approximately 5 mm or a diameter of between approximately 1 and approximately 4 mm.

## Patentansprüche

1. Feste galenische Form zur okulären Verabreichung eines Wirkstoffs, **dadurch gekennzeichnet, dass:**
- sie wenigstens einen wasserlöslichen und für eine ophthalmische Verwendung biokompatiblen Arzneimittelträger umfasst;
- sie trocken und starr ist;
- sie durch ein Verfahren erhalten wird, ausgewählt aus:
direktem Verpressen,
trockenem Verpressen,
feuchtem Verpressen,
Verpressen eines Lyophilisat,
wobei das Verpressen bei einer Temperatur von unter 45 °C durchgeführt wird, und Lyophilisierung; und
- sie eine Zerfallszeit, die den Wirkstoff in den Bindehautsack freisetzt, von niedriger als 2 Stunden aufweist.

2. Galenische Form nach Anspruch 1, **dadurch gekennzeichnet, dass** der wasserlösliche Arzneimittelträger aus wasserlöslichen und für eine ophthalmische Verwendung biokompatiblen Polymeren ausgewählt ist.

3. Galenische Form nach Anspruch 2, **dadurch gekennzeichnet, dass** der wasserlösliche biokompatible Arzneimittelträger ausgewählt ist aus Kohlenhydraten, Gummen, Natriumsalzen, Calciumsalze, Magnesiumsalzen, Maltodextrinen, Cellulosederivaten, Chitosanen, destrukturierten Stärken, partiell hydrolysierten Stärken, vernetzten Stärken, Polyvinylalkoholen und Acrylsäurepolymeren.

4. Galenische Form nach Anspruch 1 oder 2, **dadurch gekennzeichnet, dass** der wasserlösliche und für eine ophthalmische Verwendung biokompatible Arzneimittelträger ein Cellulosederivat ist, das aus Hydroxyalkylcellulosen ausgewählt ist.

5. Galenische Form nach einem der Ansprüche 1 bis 4, **dadurch gekennzeichnet, dass** der wasserlösliche und für eine ophthalmische Verwendung biokompatible Arzneimittelträger Hydroxypropylcellulose ist.

6. Galenische Form nach einem der Ansprüche 1 bis 3, **dadurch gekennzeichnet, dass** der wasserlösliche und für eine ophthalmische Verwendung biokompatible Arzneimittelträger unter Kohlenhydraten ausgewählt ist.

7. Galenische Form nach einem der Ansprüche 1 bis 6, **dadurch gekennzeichnet, dass** der wasserlösliche Arzneimittelträger in einer Konzentration zwischen etwa 40 und etwa 99,5 Gewichts-% vorliegt.

8. Galenische Form nach einem der Ansprüche 1 bis 7, **dadurch gekennzeichnet, dass** sie außerdem wenigstens einen weiteren Arzneimittelträger enthält, der ausgewählt ist aus löslichen Gleitmitteln, Zerfallsmitteln, Fließmitteln, polymeren Umhüllungsmitteln und Bindemitteln.

9. Galenische Form nach einem der Ansprüche 1 bis 8, **dadurch gekennzeichnet, dass** sie außerdem wenigstens ein lösliches Gleitmittel enthält, das aus Zuckerestern, Polyethylenglykol, Leucin und Natriumborat ausgewählt ist.

10. Galenische Form nach einem der Ansprüche 1 bis 9, **dadurch gekennzeichnet, dass** sie außerdem wenigstens ein Zerfallsmittel enthält, das aus Cellulosederivaten, Croscarmellose-Natrium, Crospovidon und Povidon ausgewählt ist.

11. Galenische Form nach Anspruch 10, **dadurch gekennzeichnet, dass** das Cellulosederivat Carboxymethylcellulose-Natrium ist.

12. Galenische Form nach einem der Ansprüche 1 bis 11, **dadurch gekennzeichnet, dass** sie außerdem wenigstens ein polymeres Umhüllungsmittel enthält, das aus thermoplastischen Cellulosederivaten ausgewählt ist.

13. Galenische Form nach einem der Ansprüche 1 bis 12, **dadurch gekennzeichnet, dass** sie außerdem wenigstens ein thermoplastisches Cellulosederivat enthält, das aus Ethylcellulose und Celluloseacetat ausgewählt ist.

14. Galenische Form nach einem der Ansprüche 1 bis 13, **dadurch gekennzeichnet, dass** sie eine pharmakologisch aktive Substanz enthält.

15. Galenische Form nach Anspruch 14, **dadurch gekennzeichnet, dass** die pharmakologisch aktive Substanz für eine vorgesehene lokale Wirkung ausgewählt ist.

16. Galenische Form nach Anspruch 14, **dadurch gekennzeichnet, dass** die pharmakologisch aktive Substanz für eine vorgesehene systemische Wirkung ausgewählt ist.

17. Galenische Form nach einem der Ansprüche 1 bis 16, **dadurch gekennzeichnet, dass** sie steril und einzeln oder als Mehrfachdosen verpackt ist.

18. Verwendung einer festen galenischen Form nach einem der Ansprüche 1 bis 17 für die Herstellung eines ophthalmischen Inserts für die okuläre Verabreichung eines Wirkstoffs durch Zerfall im Bindehautsack.

19. Verwendung nach Anspruch 18, **dadurch gekennzeichnet, dass** die Zerfallszeit unter 2 Stunden ist.

20. Ophthalmisches Insert, das durch eine galenische Form nach einem der Ansprüche 1 bis 17 gebildet wird, **dadurch gekennzeichnet, dass** die galenische Form geeignete Abmessungen für eine okuläre Verabreichung hat.

21. Ophthalmisches Insert nach Anspruch 20, **dadurch gekennzeichnet, dass** die galenische Form eine Länge von zwischen etwa 1 mm und etwa 10 mm oder einen Durchmesser, der bis zu ungefähr 10 mm reichen kann, umfasst.

22. Ophthalmisches Insert gemäß Anspruch 20 oder 21, **dadurch gekennzeichnet, dass** die galenische Form eine Länge zwischen etwa 2 mm und etwa 5 mm oder einen Durchmesser zwischen etwa 1 mm und etwa 4 mm hat.
